(19) **Europäisches Patentamt**
**European Patent Office**
**Office européen des brevets**

(11) **EP 2 101 630 B1**

(12) **EUROPEAN PATENT SPECIFICATION**

(45) Date of publication and mention
of the grant of the patent:
**13.12.2017 Bulletin 2017/50**

(21) Application number: **07860624.1**

(22) Date of filing: **28.12.2007**

(51) Int Cl.:
***A61B 1/05*** (2006.01)

(86) International application number:
**PCT/JP2007/075429**

(87) International publication number:
**WO 2008/082005 (10.07.2008 Gazette 2008/28)**

(54) **CAPSULE MEDICAL APPARATUS AND BODY-CAVITY OBSERVATION METHOD**

KAPSELFÖRMIGES MEDIZINPRODUKT UND VERFAHREN ZUR BEOBACHTUNG EINER KÖRPERHÖHLE

DISPOSITIF MÉDICAL DE TYPE CAPSULE ET PROCÉDÉ D'OBSERVATION D'UNE CAVITÉ CORPORELLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.12.2006 US 646964**
**12.01.2007 US 653168**

(43) Date of publication of application:
**23.09.2009 Bulletin 2009/39**

(73) Proprietor: **Olympus Corporation**
**Tokyo 192-8507 (JP)**

(72) Inventors:
• **KAWANO, Hironao**
**Shibuya-ku**
**Tokyo 151-0072 (JP)**
• **TAKAHASHI, Masaki**
**Shibuya-ku**
**Tokyo 151-0072 (JP)**

• **KATAYAMA, Miho**
**Shibuya-ku**
**Tokyo 151-0072 (JP)**
• **TAKIZAWA, Hironobu**
**Shibuya-ku**
**Tokyo 151-0072 (JP)**
• **SEGAWA, Hidetake**
**Shibuya-ku**
**Tokyo 151-0072 (JP)**
• **ITO, Hideo**
**Shibuya-ku**
**Tokyo 151-0072 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
**EP-A- 1 652 466      WO-A-02/095351**
**WO-A-2005/060348      JP-A- 2004 073 887**

**Description**

TECHNICAL FIELD

The present invention relates to a capsule medical apparatus capable of moving in a stable posture in liquid introduced into a subject along a flow of the liquid, for example, and capable of performing an observation and medical practice such as obtaining an image inside the subject, for example.

BACKGROUND ART

In recent years, in the field of endoscopes, there have been introduced capsule endoscopes provided with an imaging function and a wireless communication function. For the purpose of observation (examination), such a capsule endoscope is configured to sequentially image, using the imaging function, in an internal organ such as an esophagus, a stomach, or a small intestine (in a body cavity) along with peristalsis thereof, during an observation period after the capsule endoscope is taken into a patient being a subject (human body) from the mouth of the patient and until it is naturally eliminated from a living body of the patient.

It should be noted that, International Publication WO 02/95351 discloses a technique suitable for an observation of a large intestine in which a capsule endoscope is taken with liquid to float in the liquid by setting the specific gravity of the capsule endoscope to be either the same as the liquid that surrounds the capsule endoscope, or one (1), which is the same as the specific gravity of water, thereby allowing the capsule endoscope to advance speedily in the body cavity to the large intestine. In addition, while only the proximity of the capsule endoscope can be observed when the capsule endoscope sticks to a wall surface of the body cavity, it is possible to ensure a field of view for observation and to observe everything according to International Publication WO 02/95351, because the capsule endoscope is made to float in the liquid for observation.

WO 2005/060348 A relates to an in vivo device, system and a method for imaging a body lumen, typically liquid filled body lumen. The in vivo device may have a specific gravity of about 1 or a volume to weight ratio that enables it to float. The in vivo device may include a sensor, for example an image sensor, and may be attached to or set in one or more buoyant bodies. The in vivo device may be moved through the body lumen by the liquid movement in that lumen.

DISCLOSURE OF INVENTION

However, a problem has been noted that, when floating, the conventional capsule endoscope cannot move or stop along the flow of the liquid and a posture of the capsule endoscope becomes unstable, and therefore it is not possible to perform a desired observation or medical practice.

Furthermore, there has been another problem that, when a conventional capsule endoscope moves inside a wide lumen such as the large intestine, the capsule endoscope moves near a wall surface of the body cavity, and images captured by the imaging function cover in many cases only a narrow portion of areas near the wall surface of the body cavity, and thus desired images cannot be reliably obtained.

An object of the present invention is to provide a capsule medical apparatus capable of moving, floating, and stopping along the flow of liquid introduced into a subject being tested, as well as capable of taking a desired posture in a stable manner.

To solve the problems and achieve the object, a capsule medical apparatus having the features set out in independent claim 1 is provided. Further embodiments are defined by the dependent claims.

In the capsule medical apparatus, specific gravity of the capsule medical apparatus may be a proximity value of specific gravity of the liquid.

In the capsule medical apparatus, when the gravity is greater than the buoyancy, a distance between the point at which the moments are balanced and the center of buoyancy may be larger than a distance between the point at which the moments and the center of gravity, and the protruded portion may be brought into contact with an inner wall of the body cavity downward in a vertical direction of the point at which the moments are balanced.

In the capsule medical apparatus, when the gravity is smaller as compared to the buoyancy, a distance between the point at which the moments are balanced and the center of buoyancy may be smaller than a distance between the point at which the moments and the center of gravity, and the protruded portion may be brought into contact with an inner wall of the body cavity upward in a vertical direction of the point at which the moments are balanced.

In the capsule medical apparatus, either the point at which the moments are balanced may be positioned between an intersection of the straight line that intersects perpendicularly with the outer surface of the protruded portion and the protruded portion, and a center of curvature of the protruded portion at the intersection in a plane including the point at which the moments are balanced, the intersection, and the center of buoyancy, or the intersection may be positioned between the point at which the moments are balanced and the center of curvature.

In the capsule medical apparatus, an acute angle formed by the first straight line that intersects perpendicularly with the outer surface of the protruded portion and the longitudinal axis may be equal to or smaller than 80 degrees.

The capsule medical apparatus may further include a fluid resistor having a resistance to the liquid that flows in the body cavity.

In the capsule medical apparatus, the body cavity may be large intestine.

In the capsule medical apparatus, the fluid resistor may be a fin provided on a surface of the capsule housing, and capable of producing a force in a direction to move away from an inner wall surface of the body cavity in response to a flow of the liquid.

In the capsule medical apparatus, the fluid resistor may be a fin that produces a rotational motion about the longitudinal axis.

In the capsule medical apparatus, the fluid resistor may be an eccentric rotator causing eccentric rotational movement about the longitudinal axis.

In the capsule medical apparatus, the eccentric rotator may have a different amount of rotation by one fluid resistor provided at a first end of the capsule housing from that by the other fluid resistor provided at a second end of the capsule housing.

In the capsule medical apparatus, the capsule housing may include a first housing unit having an intersection of the first straight line that intersects perpendicularly with the outer surface of the protruded portion and the protruded portion, and a second housing unit having the fluid resistor. The first housing unit and the second housing unit are connected via a resilient member, energy which the fluid resistor receives is accumulated at the resilient member. The capsule medical apparatus may be separated from the inner wall surface of the body cavity using the accumulated energy.

In the capsule medical apparatus, both ends of the capsule housing in a longitudinal axis direction may form a dome shape.

In the capsule medical apparatus, the capsule housing may include an imaging system at the other end of the capsule housing, the imaging system imaging an image in the body cavity.

In the capsule medical apparatus, the center of gravity may be positioned at an eccentric position from the longitudinal axis of the capsule housing, and an imaging axis of the imaging system may be provided at an angle with respect to the longitudinal axis centering around a straight line perpendicular to a plane including the longitudinal axis and the center of gravity.

The capsule medical apparatus may further include a rectifying portion that is provided on a surface of the capsule housing and rectifies a flow of the liquid.

The capsule medical apparatus may further include a fluid resistor that is provided on a surface of the capsule housing and has the capsule medical apparatus turn about the longitudinal axis in response to a flow of the liquid.

The capsule medical apparatus may further include a magnetic body, within the capsule housing, having magnetism that is substantially perpendicular to the longitudinal axis, wherein the capsule housing turns about the longitudinal axis by rotating magnetic field applied from outside.

In the capsule medical apparatus, the protruded portion may include a flat portion, and an intersection of the first straight line that intersects perpendicularly with the outer surface of the protruded portion and the protruded portion may be at the flat portion.

In the capsule medical apparatus, the protruded portion may include a circular truncated cone plane portion, and an intersection of the first straight line that intersects perpendicularly with the outer surface of the protruded portion and the protruded portion may be at the circular truncated cone plane portion.

In the capsule medical apparatus, the capsule housing may include a body portion that is in a substantial cylindrical shape and whose center axis is parallel to the longitudinal axis, and a radius of curvature of the protruded portion at an intersection of the first straight line that intersects perpendicularly with the outer surface of the protruded portion and the protruded portion may be greater than a diameter of the substantial cylindrical shape.

In the capsule medical apparatus, the protruded portion may have a substantially oval spherical protruded shape that is connected to the substantial cylindrical shape, and the radius of curvature of the protruded portion may increase gradually toward an end in a direction of the longitudinal axis.

In the capsule medical apparatus, the protruded portion may have a substantially oval spherical protruded shape that is connected to the cylindrical shape, and the radius of curvature of the protruded portion may decrease gradually toward an end in a direction of the longitudinal axis.

In the capsule medical apparatus, the capsule medical apparatus may be in point contact with a body cavity inner wall surface at an intersection of the first straight line that intersects perpendicularly with the outer surface of the protruded portion and the protruded portion.

BRIEF DESCRIPTION OF DRAWINGS

FIG. 1 shows a schematic construction of a first embodiment of a capsule endoscope according to the present invention;

FIG. 2 is a diagram illustrating stability conditions for the capsule endoscope shown in FIG. 1 to take an inclined posture;

FIG. 3 is an illustrative diagram showing a moving state of the capsule endoscope shown in FIG. 1 in a large intestine;

FIG. 4 is a diagram illustrating the stability conditions for the capsule endoscope to take the inclined posture, when the capsule endoscope is in a point contact with an inner wall of a body cavity upward in a vertical direction;

FIG. 5 is a diagram illustrating a size of a radius of curvature that further stabilizes the capsule endoscope shown in FIG. 2;

FIG. 6 is a diagram illustrating a size of a radius of curvature that further stabilizes the capsule endoscope shown in FIG. 4;

FIG. 7 is a diagram illustrating a size of a radius of curvature that hinders the stabilization of the capsule endoscope;

FIG. 8 is a diagram illustrating an example of a point contact portion of the capsule endoscope;

FIG. 9 is a diagram illustrating an example of a point contact portion of the capsule endoscope;

FIG. 10 is a diagram illustrating an example of a point contact portion of the capsule endoscope;

FIG. 11 is a diagram illustrating an example of a point contact portion of the capsule endoscope;

FIG. 12 is a diagram illustrating an example of a capsule housing of the capsule endoscope;

FIG. 13 is a diagram illustrating an example of a capsule housing of the capsule endoscope;

FIG. 14 is a diagram illustrating an example of a capsule housing of the capsule endoscope;

FIG. 15 is a diagram illustrating an example of a capsule housing of the capsule endoscope;

FIG. 16 is a diagram illustrating an example of a capsule housing of the capsule endoscope;

FIG. 17 shows a schematic construction of a second embodiment of the capsule endoscope according to the present invention;

FIG. 18 is a block diagram showing a configuration of the capsule endoscope shown in FIG. 1;

FIG. 19 is a circuit diagram showing a configuration of a system control circuit shown in FIG. 18;

FIG. 20 shows a schematic construction of a first variant of the second embodiment of the capsule endoscope according to the present invention;

FIG. 21 shows a schematic construction of a second variant of the second embodiment of the capsule endoscope according to the present invention;

FIG. 22 shows a schematic construction of a third embodiment of the capsule endoscope according to the present invention;

FIG. 23 shows a schematic construction of a first variant of the third embodiment of the capsule endoscope according to the present invention;

FIG. 24 is a diagram illustrating a configuration of the capsule endoscope shown in FIG. 22 provided with a fin for rectification;

FIG. 25 is a diagram illustrating a configuration of the capsule endoscope shown in FIG. 23 provided with a fin for rectification;

FIG. 26 is a diagram illustrating a configuration of the capsule endoscope shown in FIG. 22 provided with a fin for turning around;

FIG. 27A is a diagram showing an outline configuration of a capsule endoscope according to a fourth embodiment of the present invention;

FIG. 27B is a diagram showing a first modification of the capsule endoscope shown in FIG. 27A obtained by disposing an imaging optical system of the capsule endoscope at both ends of the capsule housing;

FIG. 28 is a diagram schematically showing movement of the capsule endoscope shown in FIGS. 27A and 27B inside a lumen;

FIG. 29 is a diagram showing an example of desired body-cavity images captured by the capsule endoscope shown in FIGS. 27A and 27B;

FIG. 30 is a diagram showing a second modification obtained by providing a straightener with through holes in the capsule endoscope;

FIG. 31 is a diagram showing a third modification obtained by providing a straightener with fins in the capsule endoscope;

FIG. 32 is a diagram showing a fourth modification obtained by providing a straightener with grooves in the capsule endoscope;

FIG. 33 is a diagram showing a fifth modification obtained by providing a rotator with through holes in the capsule endoscope;

FIG. 34 is a diagram showing a sixth modification obtained by providing a rotator with fins in the capsule endoscope;

FIG. 35 is a diagram showing a seventh modification obtained by providing a rotator with grooves in the capsule endoscope;

FIG. 36 is a diagram showing an eighth modification obtained by providing a rotator with finned cutouts in the capsule endoscope;

FIG. 37 is a diagram showing a ninth modification obtained by providing an eccentric rotator causing eccentric movement of the capsule endoscope;

FIGS. 38A to 38I are diagrams showing modifications of a sectional shape of a through hole, a fin, a groove, or a cutout; and

FIG. 39 is a diagram showing a tenth modification obtained by providing a vibrator in the capsule endoscope.

BEST MODE(S) FOR CARRYING OUT THE INVENTION

Exemplary embodiments of a capsule medical apparatus and a body-cavity observation method according to the present invention will be described in detail below with reference to drawings. In the following exemplary embodiments, a capsule endoscope with an imaging function is explained as an example of the capsule medical apparatus. The present invention is defined by the claims, and various modifications may be implemented within the scope of the present invention.

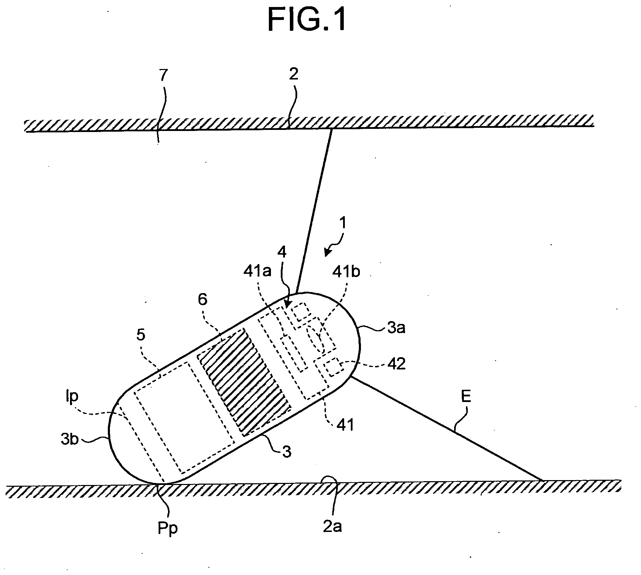

FIG. 1 shows a schematic construction of a first embodiment of a capsule endoscope according to the present invention. A capsule endoscope 1 includes a capsule housing 3 that may be introduced into a body cavity of a subject 2, an imaging optical system 4 disposed in the capsule housing 3 and capable of imaging in an anterior end direction, a circuit system unit 5 such as a control board disposed in the capsule housing 3, a circuit component, and a transmitting antenna, and a battery 6.

The size of the capsule housing 3 is such that it may be taken into the subject 2 through a mouth orifice thereof. A substantially hemispherical end cover 3a that is either transparent or translucent is elastically fitted with a body cover 3b made of a colored material that blocks visible light and is shaped like a cylinder with a bottom, thereby forming a liquid-tight external casing.

Here, the capsule endoscope 1 according to the present invention obtains an image of a subject to be imaged, for example, an inner wall of a large intestine, as an internal image of the subject. The capsule housing 3 is configured so that the specific gravity of the capsule housing 3 including components housed therein is slightly higher than that of the liquid 7 (hereinafter, sometimes referred to as "fluid 7"), and while the capsule housing 3 sinks in the liquid 7, a projection Ip of the body cover 3b comes into point contact with an inner wall surface 2a of the body cavity downward in a vertical direction at a point Pp. As a result, the capsule housing 3 takes either a desired inclined posture in which a direction of a long axis thereof is inclined with respect to a horizontal direction or a vertical posture. The liquid 7 is transmissive of a wavelength of the imaging optical system 4 of the capsule endoscope 1, and may be drunk through the mouth orifice of the subject 2. An example used in this embodiment is drinking water or intestinal lavage fluid whose specific gravity is close to one (1). It should be noted that, in this embodiment, the specific gravity of the liquid 7 may be equal to or greater than one (1).

The battery 6 is a heavy component among the components housed in the capsule endoscope 1, and disposed substantially in a central portion in the capsule housing 3. It is possible to balance the weight mainly by varying the position of the battery 6, because the battery is a heavy component.

The imaging optical system 4 is constituted by an imaging unit 41 and an illumination unit 42. The imaging unit 41 is provided, along an axis center of the capsule housing 3, with an imaging device 41a, such as a CCD or CMOS imager, that images an image of the subject to be imaged as the internal image of the subject by receiving illuminating light from the illumination unit 42 reflected on the subject to be imaged, and an imaging lens 41b that has the imaging device 41a image an optical image of the subject to be imaged. The imaging unit 41 then obtains the image of the subject to be imaged as the internal image of the subject.

The illumination unit 42 is for illuminating an imaging field E of the imaging unit 41, and realized by a plurality of light sources such as LEDs that emit illuminating light for illuminating an imaging site of the subject to be imaged via the end cover 3a. The plurality of LEDs are disposed around the imaging unit 41 centering the optical axis of the imaging unit 41 so as to cover an entire area of the imaging field E.

The imaging unit 41 is provided for the capsule endoscope housing 3 on a side not in contact with the inner wall. With this, it is possible to obtain a wider field of view in an intestinal tract having a wide space.

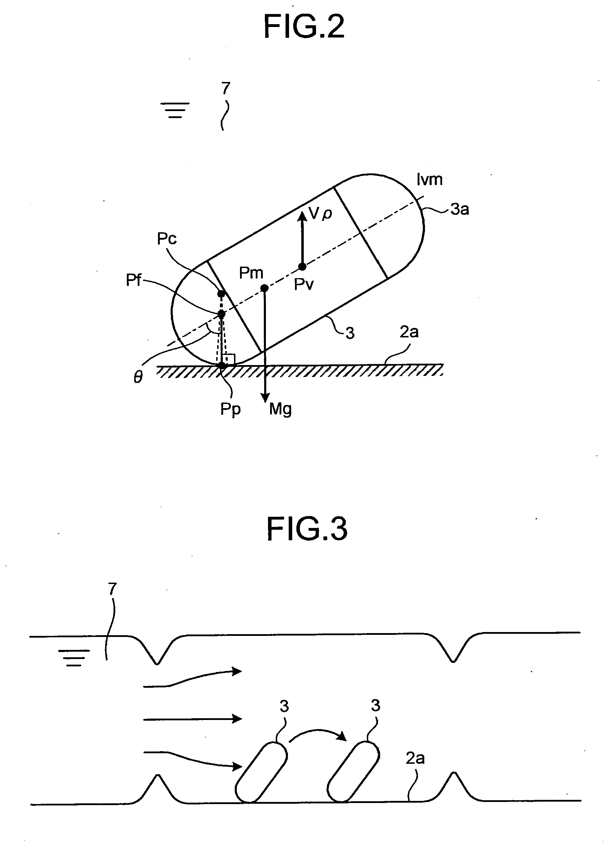

Next, referring to FIG. 2, conditions are explained under which the capsule endoscope 1 may stably take the inclined posture in a point contact state with the inner wall surface 2a of the body cavity downward in a vertical direction.

In FIG. 2, Pm represents a center of gravity of the capsule endoscope 1, and Pv represents a center of buoyancy (center of volume) of the capsule endoscope 1. Here, a straight line that passes Pm and Pv is indicated by lvm. Further, a point on lvm at which buoyant moment produced by buoyancy acting on the center of buoyancy and gravitational moment acting on the center of gravity are balanced is indicated as Pf. In other words, when Mg represents the gravity and Vp represents the buoyancy,

distance PfPv x buoyancy Vp = distance PfPm x gravity Mg is established. At this time, because the capsule endoscope 1 needs to sink to the inner wall surface 2a of the body cavity, the gravity Mg is required to be greater as compared to the buoyancy Vp. Therefore, the distance PfPm is shorter as compared to the distance PfPv.

Here, the capsule endoscope 1 includes, out of any given lines passing through the point Pf, a line that perpendicularly intersects with the projection lp of the capsule endoscope 1, and an intersection of the line with the projection lp is taken as the point Pp. The capsule endoscope 1 having the point Pp as described above may maintain its posture in the point contact state at the point Pp with the inner wall surface 2a of the body cavity. Moreover, when the meeting of a straight line connecting the point Pp and the point Pf and the straight line lvm forms an acute angle $\theta$, the long axis of the capsule keeps the inclined posture at an angle of 90° - $\theta$ with the horizontal plane. It should be noted that, while the long axis of the capsule endoscope 1 and the line lvm match each other in this example, the present invention is not limited to this example, and the same principle is established when the long axis and the line lvm do not match as in a third embodiment which will be described later.

Further, a central position Pc is positioned along the straight line connecting the point Pp and the point Pf, and the central position Pc is a central position of curvature in a plane including the points Pv, Pf, and Pp at a surface of the point Pp. Therefore, a distance between the point Pp and the point Pc is a radius of curvature.

Although not shown here, when the moment due to the buoyancy produced in the capsule endoscope 1 is sufficiently greater than the moment due to the gravity, the posture of the capsule endoscope 1 is stabilized at an angle at which the line lvm becomes vertical (i.e., the moment due to the buoyancy and the moment due to the gravity are balanced). At this time, the moments centering the point Pf become zero (0) both for the moment due to the buoyancy and the moment due to the gravity.

When the capsule endoscope 1 can maintain the inclined posture or the vertical posture with respect to the inner wall surface 2a of the body cavity in a stable manner, a fluid resistance against the liquid 7 becomes greater as shown in FIG. 3, and the capsule endoscope 1 can easily move along the flow of the liquid 7 because the capsule endoscope 1 is in the point contact state with the inner wall surface 2a of the body cavity. Further, because the capsule can float easily, it is possible to obtain an image at a center of a lumen and improve observability. In this case, the capsule endoscope 1 maintains substantially the inclined posture or the vertical posture in a stable manner, it is possible to obtain an image of an entire or a desired wall in the body cavity. Note that, when the capsule endoscope 1 takes a horizontal posture and is in a line contact state with the inner wall surface 2a of the body cavity, it is not possible to obtain sufficient fluid resistance against the liquid 7, thereby decreasing mobility and flotation of the capsule endoscope 1. In particular, in the body cavity having a large lumen diameter such as a large intestine, the flow of the liquid 7 is faster around the lumen center than near the lumen wall, and therefore, when the capsule endoscope 1 is in the line contact state with the wall, the mobility and the flotation are decreased.

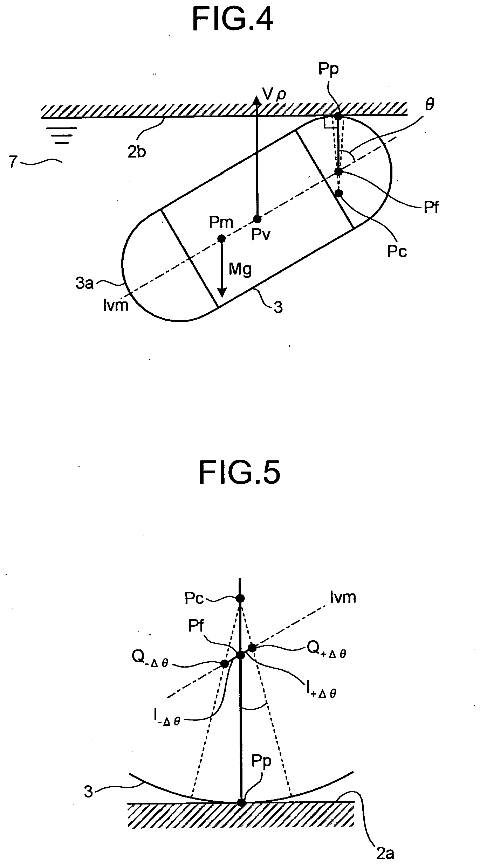

Here, the capsule endoscope 1 shown in FIG. 2 is in point contact with the inner wall surface 2a of the body cavity downward in the vertical direction. However, as shown in FIG. 4, the capsule endoscope 1 may be in point contact with the inner wall surface 2b of the body cavity upward in the vertical direction.

The stability conditions of the capsule endoscope 1 in this case are different from those of the capsule endoscope 1 shown in FIG. 2 in that, firstly, a gravity Mg of the capsule endoscope 1 is smaller as compared to a buoyancy Vp of the capsule endoscope 1. Further, based on the balance of the moments, the distance PfPm is longer than the distance PfPv. Moreover, when the moment due to the gravity produced in the capsule endoscope 1 is sufficiently greater than the moment due to the buoyancy, the posture of the capsule endoscope 1 is stabilized at an angle at which the line lvm becomes vertical (i.e. the moment due to the buoyancy and the moment due to the gravity are balanced). At this time, the moments centering the point Pf become zero (0) both for the moment due to the buoyancy and the moment due to the gravity.

In the meantime, although the above mentioned capsule endoscopes 1 as shown in FIG. 2 or FIG. 4 can maintain the point contact state respectively with the inner wall surface 2a or 2b of the body cavity, it is possible to maintain the inclined posture or the vertical posture even more stably by either positioning the point Pf between the points Pp and Pc, or positioning the points Pp between the points Pf and Pc.

FIG. 5 shows a proximity of the point contact portion of the capsule endoscope shown in FIG. 2, and in the above mentioned state in which the moments of the gravity and the buoyancy are balanced, it is possible to stabilize the posture like a tumble doll weighted at the base, by either positioning the point Pf between the points Pp and Pc, or positioning the points Pp between the points Pf and Pc, even when a disturbance (-$\Delta\theta$, +$\Delta\theta$) is added.

In FIG. 5, when there is a displacement of -$\Delta\theta$, that is, when the line lvm of the capsule endoscope 1 shifts in the vertical direction, a displacement in moment $\alpha$ (moment in the vertical direction - moment in the horizontal direction) becomes as follows with a displacement in length $l_{-\Delta\theta}$ on the line lvm.

$$\alpha = (PvPf + l_{-\Delta\theta}) \times V\rho - (PmPf + l_{-\Delta\theta}) \times Mg$$

$$= l_{-\Delta\theta} \times (V\rho - Mg)$$

$$< 0$$

Therefore, a moment is produced in a direction in which the acute angle $\theta$ increases, i.e. the horizontal direction, and negates the displacement of $-\Delta\theta$. In other words, a restoring force is produced.

Similarly, when there is a displacement of $+\Delta\theta$ (displacement in the horizontal direction), the displacement in moment $\alpha$ is as follows.

$$\alpha = (PvPf - l_{+\Delta\theta}) \times V\rho - (PmPf - l_{+\Delta\theta}) \times Mg$$

$$= l_{+\Delta\theta} \times (Mg - V\rho)$$

$$> 0$$

In this case also, the displacement of $+\Delta\theta$ is negated, i.e. the restoring force is produced. Therefore, the restoring force is produced when one of the disturbances $-\Delta\theta$ and $+\Delta\theta$ is produced, and the inclined posture of the capsule endoscope 1 may be kept even more stably.

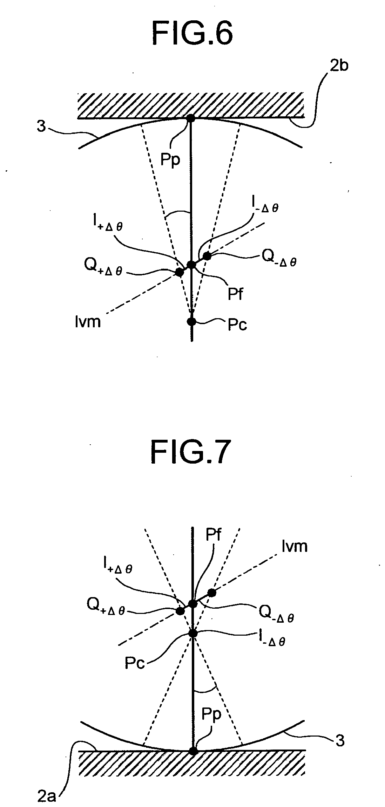

Furthermore, the same applies when the capsule endoscope 1 is in point contact with the inner wall surface 2b of the body cavity upward in the vertical direction as shown in FIG. 4. That is, when there is the displacement of $-\Delta\theta$ (displacement in the vertical direction) in FIG. 6, the displacement in moment $\alpha$ is as follows.

$$\alpha = (PvPf + l_{-\Delta\theta}) \times Mg - (PmPf + l_{-\Delta\theta}) \times V\rho$$

$$= l_{-\Delta\theta} \times (Mg - V\rho)$$

$$< 0$$

Therefore, a moment is produced in a direction in which the acute angle $\theta$ increases, i.e. the horizontal direction, and negates the displacement of $-\Delta\theta$. In other words, a restoring force is produced.

Similarly, when there is a displacement of $+\Delta\theta$ (displacement in the horizontal direction), the displacement in moment $\alpha$ is as follows.

$$\alpha = (PvPf - l_{+\Delta\theta}) \times Mg - (PmPf - l_{+\Delta\theta}) \times V\rho$$

$$= l_{+\Delta\theta} \times (V\rho - Mg)$$

$$> 0$$

In this case also, the displacement of $+\Delta\theta$ is negated, i.e. the restoring force is produced. Therefore, the restoring force is produced when one of the disturbances $-\Delta\theta$ and $+\Delta\theta$ is produced in a case in which the capsule endoscope 1 is in point contact with the inner wall surface 2b of the body cavity upward in the vertical direction, and the inclined posture of the capsule endoscope 1 may be kept even more stably.

It should be noted that as shown in FIG. 7, when the point Pc is positioned between the points Pf and Pp, the displacement in moment $\alpha$, acts in a direction that amplifies the disturbance when the disturbance occurs, because the sign in $l_{-\Delta\theta}$ or $l_{+\Delta\theta}$ (direction) is inverted.

Also, as the radius of curvature PpPc becomes greater, a range in which the point Pf may be positioned becomes larger. In addition, when a condition for stabilization with respect to a disturbance in $\Delta\theta$ is satisfied by the points Pf, Pp, and Pc, values for $l_{-\Delta\theta}$ and $l_{+\Delta\theta}$ when the disturbance occurs become greater as the radius of curvature PpPc becomes greater, and accordingly, a value for the displacement in moment $\alpha$ becomes greater. In other words, when the disturbance occurs, the restoring force to restore the posture of the capsule endoscope 1 becomes greater and thus the capsule endoscope 1 becomes more stabilized.

Here, the capsule endoscope 1 as described above has the body cover 3b that is substantially hemispherical, and therefore the capsule endoscope 1 may come into point contact with the inner wall surface 2a or 2b of the body cavity. In the first embodiment according to the present invention, the capsule endoscope 1 may be provided with at least a protruding portion with which the capsule endoscope 1 may come into point contact with the inner wall surface 2a or 2b of the body cavity when the capsule endoscope 1 takes the inclined posture or the vertical posture.

Further, it is desirable that the acute angle formed by the meeting of the straight line connecting the point Pp and the point Pf with a center line of the long axis of the capsule endoscope 1 is equal to or smaller than 80°. At this time, the capsule endoscope 1 takes the inclined posture with the direction of its long axis forming an angle of 10° or greater with the horizontal direction. With this, the capsule endoscope 1 may easily receive the fluid resistance.

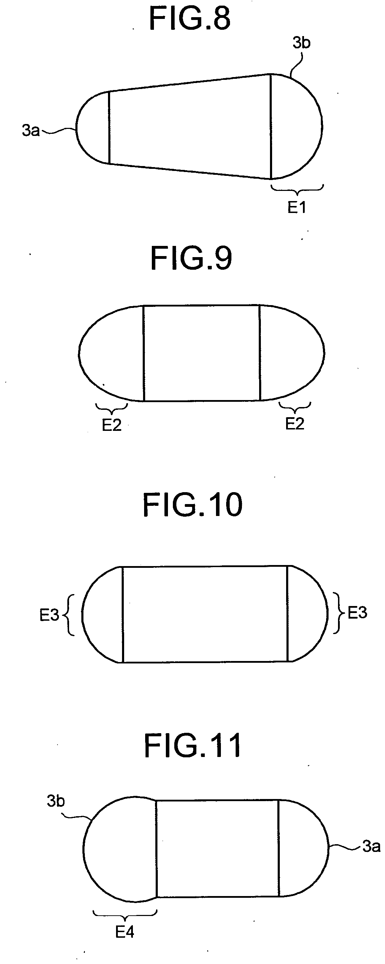

FIG. 8 shows a variant of an appearance of the capsule endoscope 1, and the radius of curvature for the body cover 3b is made greater than the radius of curvature for the end cover 3a. As a result, the capsule endoscope shown in FIG. 8 has a larger radius of curvature for a point contact region E1, and it is possible to take the inclined posture or the vertical posture in a stable manner. Further, it is desirable that the radius of curvature for the point contact region E1 is greater than the diameter of the body cover.

With the capsule endoscope shown in FIG. 9, a radius of curvature for a substantially hemispherical or substantially oval spherical shaped body that may be the body cover 3b is made greater than a diameter of a body part, and the part at which this radius of curvature is made greater is set to be a point contact region E2. Further, it is desirable that the radius of curvature for the point contact region E2 is greater than the diameter of the body cover.

In addition, with the capsule endoscope shown in FIG. 10, a radius of curvature for a tip portion of a substantially hemispherical or substantially oval spherical shaped body that may be the body cover 3b is made greater than a diameter of a body part, and the part at which this radius of curvature is made greater is set to be a point contact region E3. This capsule endoscope is suitable to take the vertical posture.

Further, with the capsule endoscope shown in FIG. 11, the body cover 3b of a substantially hemispherical shape which is greater than the diameter of the body portion is formed to obtain a point contact region E4 obtaining a large radius of curvature.

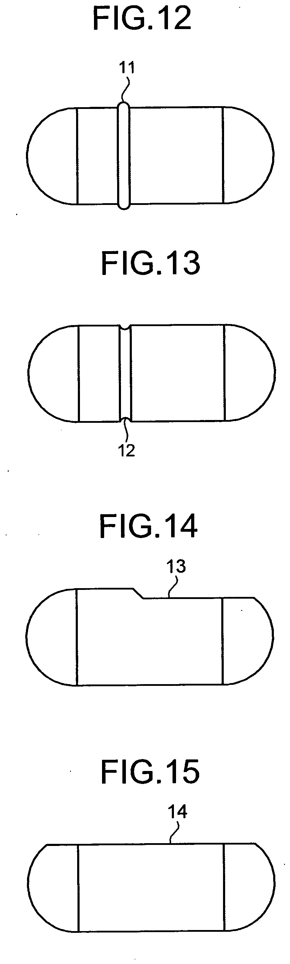

Moreover, in this first embodiment, as shown in FIG. 12 and FIG. 13, a projection 11 or a groove 12 may be formed on the body portion. This is because it does not affect the point contact between the capsule endoscope 1 and the inner wall surface 2a or 2b of the body cavity.

Further, a D-cut or the like may be formed at a part of the body portion and the end cover 3a as shown in FIG. 14, and a D-cut or the like may be formed on a side in a long axis direction as shown in FIG. 15, because, in either case, the fact that a point contact region is formed or the D-cut is formed does not affect the point contact.

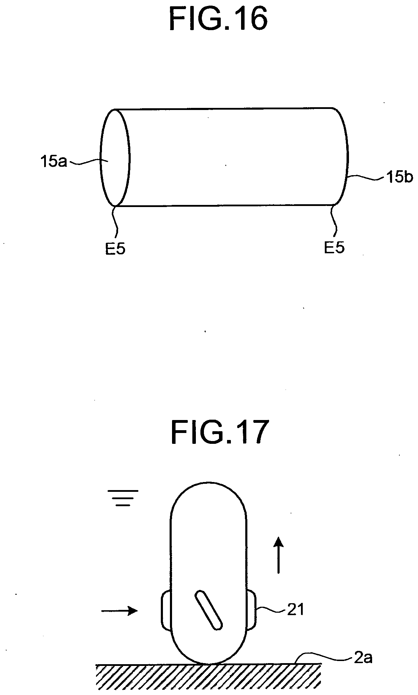

In addition, in the above described first embodiment, the point contact portion is substantially hemispherical. However, it is not limited to such an example, and the capsule endoscope may be cylindrical, and peripheral portions of both end surfaces 15a and 15b may be set to be a point contact region E5 as shown in FIG. 16. In this case, smooth chamfering may be performed in order to make a radius of curvature of the point contact region E5 larger.

In this first embodiment, the fluid resistance due to the liquid flowing through the body cavity is made greater by having the capsule endoscope take the inclined posture or the vertical posture, and a contact resistance is made smaller by bringing the capsule endoscope into point contact with the inner wall surface of the body cavity. Accordingly, it is possible to have the capsule endoscope smoothly move along the flow of the liquid. In particular, the fluid resistance from the fast flow of the liquid in the proximity of the center of the body cavity is easily obtained, and therefore it is possible to move easily. Further, the capsule endoscope itself may keep the stable posture when the capsule endoscope moves or stops, and therefore, it is possible to stably obtain a desired image inside the subject. Moreover, it is possible to obtain the image in the center of the lumen because the capsule may float easily, and accordingly the observability can be improved.

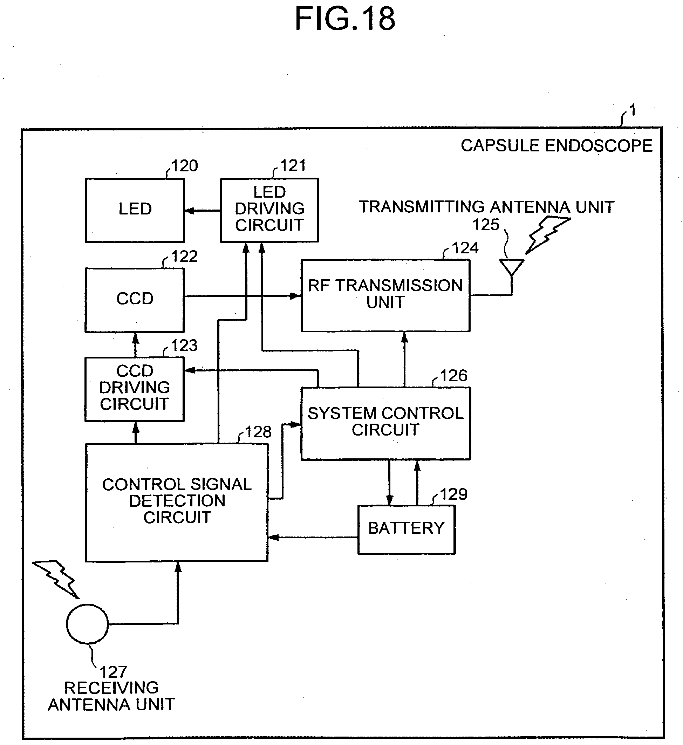

It should be noted that, as shown in a block diagram of FIG. 18, the capsule endoscope 1 is provided with, for example, a light emitting diode (LED) 120 as irradiation means for irradiating a subject site in the body cavity in the subject, an LED driving circuit 121 as first drive means that controls a driving state of the LED 120, an imaging device (hereinafter referred to as CCD) 122, such as a CCD imager or a C-MOS imager, for example, as obtaining means that images an object in the body cavity that is light reflected from the region irradiated by the LED 120 (information in the subject), a CCD driving circuit 123 as first drive means that controls a driving state of the CCD 122, an RF transmission unit 124 that modulates image signals that have been imaged into RF signals, and a transmitting antenna unit 125 as wireless transmission means that wirelessly transmits the RF signals output from the RF transmission unit 124.

Moreover, the capsule endoscope 1 is provided with a system control circuit 126 that controls operations of the LED driving circuit 121, the CCD driving circuit 123, and the RF transmission unit 124, and with this, the capsule endoscope 1 may operate so that, while the capsule endoscope 1 is introduced in the subject, image data of the subject site irradiated by the LED 120 is obtained by the CCD 122.

Thereafter, the obtained image data is converted into the RF signals by the RF transmission unit 124, and transmitted outside the subject via the transmitting antenna unit 125.

Further, the capsule endoscope 1 is provided with a receiving antenna unit 127 as wireless reception means capable of receiving wireless signals transmitted from a communication device that is external to the subject and not shown in the figure, a control signal detection circuit 128 that detects control signals of a predetermined input level (level of intensity in reception, for example) out of the signals received by the receiving antenna unit 127, and a battery 129 that supplies power to the system control circuit 126 and the control signal detection circuit 128.

The control signal detection circuit 128 detects contents of the control signals, and outputs the control signals, as necessary, to the LED driving circuit 121, the CCD driving circuit 123, and the system control circuit 126. The system control circuit 126 has a function of distributing the driving power supplied from the battery 129 to the other components (means for performing functions).

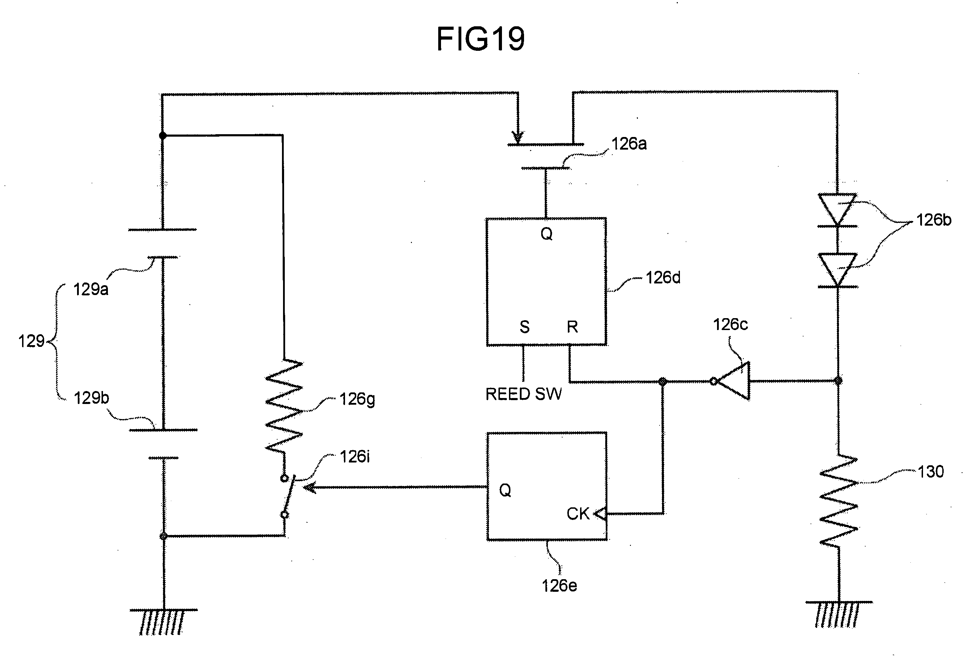

Moreover, FIG. 19 is a circuit diagram showing a circuit configuration of the system control circuit 126. In FIG. 19, the battery 129 is constituted by one or more (two in the first embodiment), for example, button type silver oxide batteries 129a and 129b.

The system control circuit 126 is provided with a FET (field effect transistor) 126a whose source terminal is connected to the battery 129, a diode 126b connected to a drain terminal of the FET 126a, a NOT circuit 126c connected to an output terminal of the diode 126b, and a flip flop 126d that is reset (R) by an output from the NOT circuit 126c and outputs (Q) to a gate terminal of the FET 126a.

The output from the diode 126b is connected to a in-capsule function performing circuit 130, and the flip flop 126d is set (S) by an input from a reed switch that detects an external magnetizing field.

It should be noted that in this first embodiment, a switching element, for example, may be used instead of a transistor such as an FET. Further, in this first embodiment, an imaging function, a illumination function, and a wireless function (partial) provided for the capsule endoscope 1 are referred to collectively as function performing means for performing predetermined functions. Specifically, the components excluding the system control circuit 126, the receiving antenna unit 127, and the control signal detection circuit 128 are included in the function performing means for performing the predetermined functions, and also referred to as the in-capsule function performing circuit 130 as needed.

Moreover, the system control circuit 126 is provided with a flip flop 126e to which an output of the NOT circuit 126c is input (CK), a resistance 126g connectable to the button type batteries 129a and 129b, and a switching element 126i.

An operation of the switching element 126i is controlled by the NOT circuit 126c and the flip flop 126e so as to be in an off state while the button type batteries 129a and 129b supplies the driving power to the in-capsule function performing circuit 130, and switched to an on state when the supply of the driving power to the in-capsule function performing circuit 130 stops.

Specifically, the switching element 126i is switched to the on state by the output (Q) from the flip flop 126e, and connects the button type batteries 129a and 129b with the resistance 126g to have the power charged in the button type batteries 129a and 129b be exhausted.

Next, an operation of the capsule endoscope 1 is described using the circuit diagram FIG. 19. In FIG. 19, for example, the capsule endoscope 1 before introduced into the subject includes a reed switch therein that can be turned on and off by an external magnetizing field, and stored in a package that includes a permanent magnet for supplying the external magnetizing field. In this state, the capsule endoscope 1 is not driven.

Next, when it is taken in, as the capsule endoscope 1 is taken out of the package, the capsule endoscope 1 is separated from the permanent magnet of the package, and the capsule endoscope 1 is not affected by the magnetic force, and the flip flop 126d is set (S) by the input from the reed switch. Once set, the flip flop 126d outputs (Q) to the gate terminal of the FET 126a, and a current flows between the source and drain terminals of the FET 126a by this output (Q), and the power from the button type batteries 129a and 129b is supplied to the in-capsule function performing circuit 130 via the diode 126b.

Here, when a voltage supplied from the button type batteries 129a and 129b is A, and voltages consumed at the FET 126a and the diode are respectively B and C, a voltage to be supplied to the in-capsule function performing circuit 130 is A - (B + C) = X. Further, the NOT circuit 126c is set with a midpoint potential Y as a threshold, and when the voltage X is greater than the midpoint potential Y, i.e. (voltage X) > (midpoint potential Y), it is not output from the NOT circuit 126c, and the switch element 126i is switched to the off state.

Moreover, when the voltage X is equal to or smaller than the midpoint potential Y, i.e. (voltage X) ≤ (midpoint potential Y), the flip flop 126d is reset by the output from the NOT circuit 126c, and the output from the NOT circuit 126c is input to the flip flop 126e. Then, when the flip flop 126d is reset, the current does not flow between the source and drain terminals, and the driving power is not supplied to the in-capsule function performing circuit 130.

Furthermore, when the output from the NOT circuit 126c is input, the flip flop 126e outputs (Q), and switches the switch element 126i to the on state.

The button type batteries 129a and 129b and the resistance 126g are connected by this switching operation, and the power charged in the button type batteries 129a and 129b is exhausted by the resistance 126e.

A second embodiment according to the present invention is described below. In the above described first embodiment, the mobility and the flotation of the capsule endoscope are facilitated by increasing the fluid resistance that the capsule endoscope itself receives. In this second embodiment, a function of further increasing the fluid resistance is provided for the capsule endoscope.

FIG. 19 is a circuit diagram showing a schematic configuration of the capsule endoscope of the second embodiment according to the present invention. The capsule endoscope shown in FIG. 17 is in point contact with the inner wall surface 2a of the body cavity downward in the vertical direction, and takes the vertical posture in which the long axis aligns with the vertical direction. This capsule endoscope is provided with a fin 21 around the body portion for turning the capsule endoscope about the long axis in response to the flow of the liquid 7.

In addition that the capsule endoscope as a whole increases the fluid resistance in response to the flow of the liquid, this capsule endoscope is configured to provide lift by the fin 21 with help of the fluid resistance so that the capsule endoscope is separated away from the inner wall surface 2a of the body cavity. With this configuration, the capsule endoscope becomes easier to move in response to the flow of the liquid.

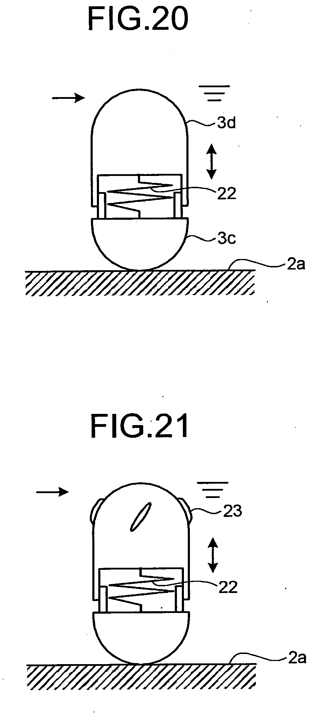

Further, FIG. 20 shows a schematic construction of the capsule endoscope as a variant of the second embodiment of according to the present invention. The capsule endoscope shown in FIG. 20 is separated into a partial housing 3c having the point contact portion and a housing body 3d that is on the end cover side, and the partial housing 3c and the housing body 3d are connected by a spring member 22 as a resilient member. In addition, this capsule endoscope takes the vertical posture similarly to the capsule endoscope shown in FIG. 17.

In addition that this capsule endoscope receives the fluid resistance on the entire capsule endoscope, the housing body 3d moves, for example, downward in the vertical direction by the fluid resistance, energy increases in compression of the spring member 22, then the housing body 3d moves upward in the vertical direction by repulsive force due to extension of the spring member 22, and the partial housing 3c moves upward in the vertical direction by inertia force at this time, thereby releasing the point contact state and thus improving the mobility and the flotation of the capsule endoscope.

FIG. 21 shows a construction combining the above configurations shown in FIG. 19 and FIG. 20. However, a fin 23 does not provide lift upward in the vertical direction, but downward in the vertical direction. Consequently, it is possible to further increase the compression of the spring member 22, and the release of the point contact state of the capsule endoscope is even more facilitated.

In this second embodiment, the fluid resistance is further increased in addition to the fluid resistance that the capsule endoscope itself receives to release the point contact state, the mobility and the flotation of the capsule endoscope along the flow of the fluid are further improved.

A third embodiment according to the present invention is described below. In the first and the second embodiments, the capsule endoscope keeps the inclined posture or the vertical posture while maintaining the point contact state with the inner wall surface of the body cavity. However, in this third embodiment, the capsule endoscope is configured to obtain a stable image in the body cavity in a direction of the lumen axis utilizing the stable inclined posture.

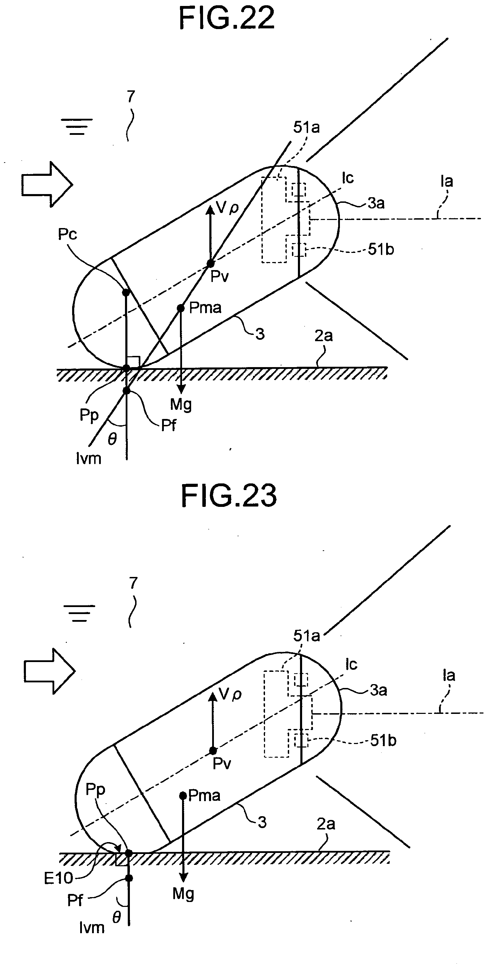

FIG. 22 shows a schematic construction of the capsule endoscope of the third embodiment according to the present invention. In this capsule endoscope, the center of gravity Pma is provided at an eccentric position from a long axis center line 1c, and furthermore this capsule endoscope is provided so that an optical axis 1a of the imaging optical system provided on the end cover side and including an imaging unit 51a and an illumination unit 51b is at an angle with respect to the long axis center line 1c of the capsule endoscope. It should be noted that the optical axis 1a is provided at an angle with respect to the long axis in a direction of eccentricity of the center of gravity, centering a straight line perpendicular to a plane including the long axis and the center of gravity.

This capsule endoscope takes the inclined posture in which the direction of eccentricity of the center of gravity Pma from the long axis center line 1c always faces downward in the vertical direction. Accordingly, the optical axis 1a of the imaging optical system is directed always at the same angle to the surroundings from the inner wall surface 2a of the body cavity downward in the vertical direction. Moreover, because the capsule endoscope as a whole, similarly to the first embodiment, easily receives the fluid resistance, this capsule endoscope is directed toward the direction of the flow of the fluid, i.e. downstream. As a result, this capsule endoscope is allowed to obtain an image in the body cavity always substantially at the same angle and facing the downstream of the fluid, as well as determination of the obtained image in the body cavity becomes easier because the capsule endoscope does not turn about the long axis. Further, although not shown, when the direction of angle of the optical axis 1a to the long axis center line is opposite to the direction of eccentricity, the same effect may be obtained in a situation in which the body cavity runs in the vertical direction.

Although the capsule endoscope shown in FIG. 22 and the inner wall surface 2a of the body cavity are in point contact, this point contact may be in plane contact as shown in FIG. 23. However, the capsule endoscope takes the inclined posture. In other words, the capsule endoscope is provided with a flat region E10 at a portion on the side of the body cover. Other configuration is the same as the capsule endoscope shown in FIG. 22. However, the portion where the flat region E10 is provided is provided in the direction of eccentricity of the center of gravity Pma from the long axis center line 1c. In the capsule endoscope shown in FIG. 23, because the flat region E10 is in plane contact with the inner wall surface 2a of the body cavity, the inclined posture is stabilized and it is possible to obtain a desired image in the body cavity.

Further, although not shown, it is possible to achieve the same effect by configuring such that the body cover portion of the capsule endoscope includes a flat portion of a circular truncated cone, and a bus line portion thereof is brought into line contact with an intestine wall, and the desired image of the body cavity may be stably obtained. In addition, because the intestine wall is brought into line contact with the capsule endoscope as well as because the capsule endoscope takes the inclined posture, similarly to the point contact state, the capsule endoscope 1 receives the fluid resistance easily.

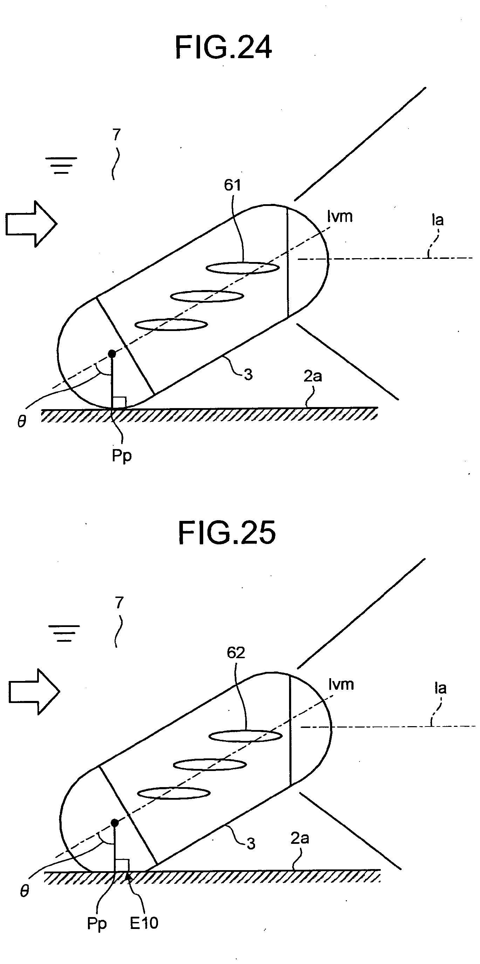

Moreover, as shown in FIG. 24 and FIG. 25, fins 61 and 62 that rectify the flow of the fluid 7 may be provided on outer surfaces of the capsule endoscopes shown in FIG. 22 and FIG. 23, respectively. The fins 61 and 62, when the capsule endoscope takes the inclined posture, rectify the flow along the flow direction of the fluid 7, and ensure the maintenance of the inclined posture.

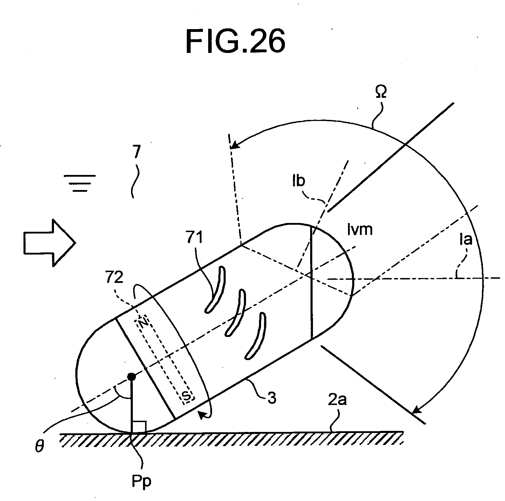

In addition, a fin 71 as shown in FIG. 26 may be provided so that the capsule endoscope turns about the long axis center line in response to the flow of the fluid 7. With the turning of the capsule endoscope about the long axis center line, the optical axis 1a rotates and a solid angle $\Omega$ of a substantial imaging area increases, thereby obtaining a wide area of the image in the body cavity.

It should be noted that, as shown in FIG. 26, it is possible to provide a magnet 72 that perpendicularly intersects with the long axis center line to apply a rotating magnetic field from outside, thereby having the capsule endoscope turn.

In this third embodiment, because the center of gravity is decentered so that the capsule endoscope does not turn about the long axis center line when the capsule endoscope takes the inclined posture, it is possible to set the optical axis of the imaging optical system at an angle with the long axis center line, and the image in the body cavity in the desired direction may be obtained stably.

Although, in the first to the third embodiments, the specific gravity of the capsule endoscope is explained to be slightly smaller or larger than the specific gravity of the liquid, it is not limited to these embodiments. The relation between the specific gravity of the capsule endoscope and the specific gravity of the liquid may be set so that capsule endoscope may move easily, considering the fluid resistance that the capsule endoscope receives and the contact state such as friction with the wall surface.

Further, not limited to the capsule endoscope, the same effect can be obtained with capsule medical apparatuses such as an ultrasonic capsule that is provided with an ultrasonic inspection device and obtains an ultrasonic image in the subject and a sensor capsule provided with a sensor for detecting a specific substance, PH, or a pressure in the subject. Here, because the ultrasonic capsule easily floats by the flow in the body cavity, it is possible to obtain the ultrasonic image in the center of the body cavity, thereby improving the observability.

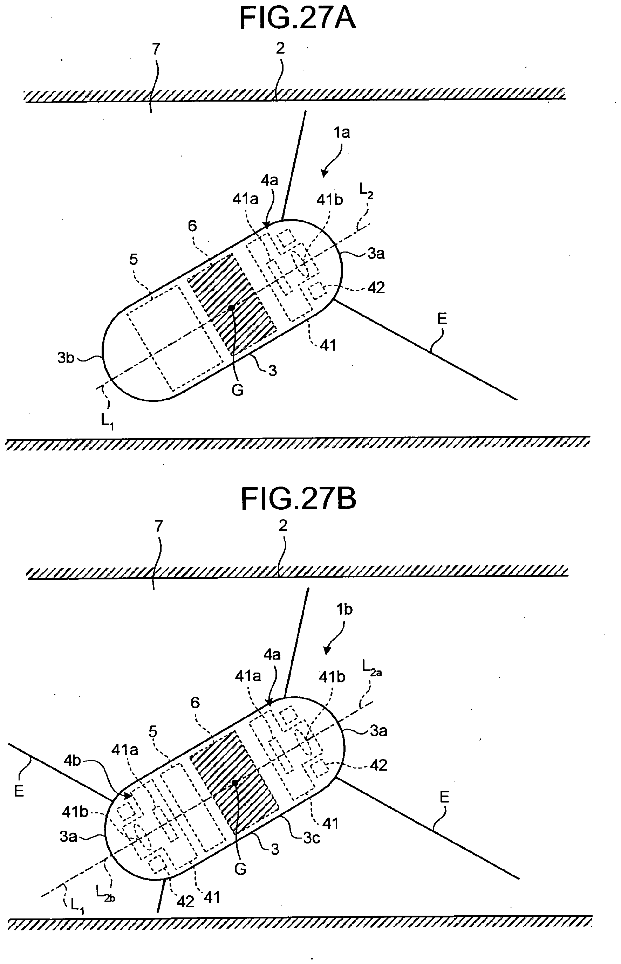

A fourth embodiment according to the present invention is described below. FIG. 27A is a diagram showing an outline configuration of a capsule endoscope, which is the fourth embodiment of the capsule medical apparatus according to the present invention. A capsule endoscope 1a includes a capsule housing 3 insertable into a body cavity of a subject 2, an imaging optical system 4a that is disposed inside the capsule housing 3 and can capture images in a front end direction, circuit systems 5 such as a control board, circuit components, and a transmitting antenna disposed inside the capsule housing 3, and a battery 6.

The capsule housing 3 is of such a size that it can be swallowed into a body through an oral cavity of the subject 2, and forms an exterior case that seals an inner part fluid-tightly by elastically fitting an approximately hemispherical end cover 3a having transparency or translucency and a closed-end cylindrical trunk cover 3b made of colored material to which visible light is not transparent.

Here, the capsule endoscope 1a of the present invention is used to obtain, for example, an inner wall of the large intestine as in-vivo images, and the capsule housing 3 has a specific gravity including incorporated components thereof roughly equal to that of a fluid 7 and a center of gravity G is located approximately in a volume center. As a result, the capsule endoscope 1a in the fluid 7 is unstable in its posture and position. The fluid 7 is a fluid that can be swallowed through the oral cavity of the subject 2 and is transparent to wavelengths of the imaging optical system 4a of the capsule endoscope 1a and, in the present embodiment, potable water or clyster whose specific gravity is roughly equal to one (1) is exemplarily used. However, the specific gravity may be greater than one (1) in the present embodiment.

The battery 6 is a heavy load among components of the capsule endoscope 1a and is disposed in approximately a central part of the capsule housing 3, and because it is heavy load, a weight balance can be maintained mainly by changing its position.

The imaging optical system 4a includes an imaging unit 41 and an illumination unit 42. The imaging unit 41 includes an imaging device 41a such as a CCD or CMOS imager that captures an image of an object by receiving a reflected light of the object by an illumination light of the illumination unit 42 as an intra-subject image and an imaging lens 41b for forming an image of the object on the imaging device 41a on an axial center of the capsule housing 3 to obtain an image of an object as an intra-subject image.

The illumination unit 42 is used for illuminating a imaging field E of the imaging unit 41 and is realized by a plurality of light sources, for example, LEDs radiating an irradiation light for illuminating an imaging region of an object via the end cover 3a. The plurality of LEDs are disposed around the imaging unit 41 with respect to an optical axis center of the imaging unit 41 so that the entire imaging field E is covered.

Meanwhile, a longitudinal axis center line L1 of the capsule endoscope 1a and an optical axis L2 of the imaging optical system coincide with each other in the present embodiment.

As already described above, the capsule endoscope 1a in the fluid 7 is unstable in its posture and position. As a result, the capsule endoscope 1a becomes, on the other hand, more susceptible to an influence of the flow of the fluid 7 and easier to move.

FIG. 27B is a diagram showing the configuration in which the imaging optical systems 4a, 4b are disposed at both ends of the capsule housing 3 in the capsule endoscope, which is the embodiment of the capsule medical apparatus according to the present invention. The capsule housing 3 forms an exterior case that seals an inner part fluid-tightly by elastically fitting approximately-hemispherical end covers 3a each having transparency or translucency respectively on both ends of an approximately-cylindrical trunk cover 3c made of colored material to which visible light is not transparent. In the present embodiment, the longitudinal axis center line L1 of a capsule endoscope 1b, an optical axis L2a of the imaging optical system 4a, and an optical axis L2b of an imaging optical system 4b coincide with each other.

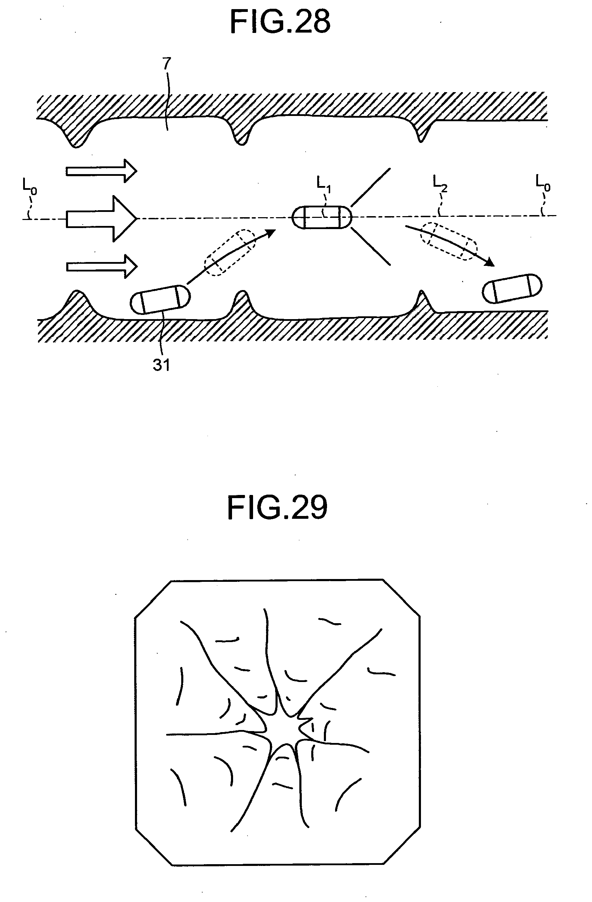

In a lumen of the large intestine for example, as shown in FIG. 28, the flow of the fluid 7 is fast near a luminal central axis $L_0$ and slow near a luminal wall surface. Thus, a capsule endoscope 31 positioned near the wall surface is less susceptible to the flow of the fluid 7, but since the capsule endoscope 31 has a specific gravity roughly equal to that of the fluid 7 and its center of gravity G is located approximately in the volume center, the capsule endoscope 31 is unstable and susceptible also to a slow flow of the fluid 7 and thus can move easily. The flow of the fluid 7 may be a flow caused passively for example by gravity, peristaltic movement, and segmentation, or a flow caused actively for example by manual pressure, purgative intake, and postural change.

If the capsule endoscope 31 moves away from the luminal wall surface under an influence of the flow of the fluid 7, the capsule endoscope 31 approaches the luminal central axis $L_0$ while moving along the flow due to a difference between a flow rate of a vicinity of the luminal central axis $L_0$ and that of the luminal wall surface. After approaching the luminal central axis $L_0$, the capsule endoscope 31 moves in a posture in which the longitudinal axis center line $L_1$ is in line with the luminal central axis $L_0$ because the capsule endoscope 31 has an approximately cylindrical shape stretching in the longitudinal axis direction and both ends have the approximately hemispherical shape. The capsule endoscope 31 is, so to speak, centered in the lumen and the optical axis $L_2$ also moves in the direction of the luminal central axis $L_0$. The capsule endoscope 31 near the luminal central axis $L_0$ where the flow rate of the fluid 7 is fast is stable in its posture and position.

As a result, the capsule endoscope 31 can capture desired body-cavity images that can command a panoramic view of the luminal wall surface along the direction of the luminal central axis $L_0$, that is, from a downstream direction or an upstream direction of the fluid 7. As shown in FIG. 29, the luminal central axis $L_0$ is positioned in the center of a captured body-cavity image.

If a difference between a flow rate of the luminal central axis $L_0$ and a vicinity of the luminal wall surface disappears, as shown in FIG. 28, the capsule endoscope 31 moves away from the vicinity of the luminal central axis $L_0$ and becomes unstable.

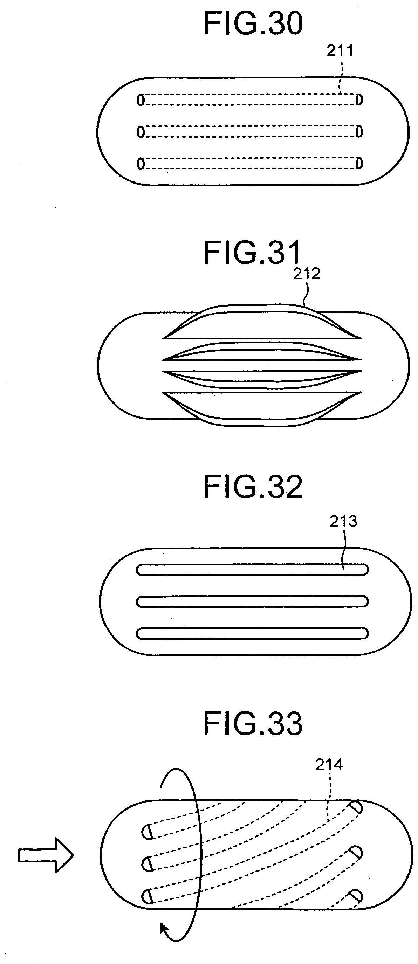

The capsule endoscopes 1a, 1b, and 31 described above are stable near the luminal central axis $L_0$ where the flow rate of the fluid 7 is fast because the capsule housing itself has an elongated shape, but in order to further increase stability, a plurality of through holes 211 along the longitudinal axis direction of the capsule housing 3 shown in FIG. 30 may be provided to straighten the fluid 7. Through straightening of the fluid 7 by the through holes 211, the direction of flow of the fluid 7 and the longitudinal axis direction of the capsule endoscope 1 coincide with each other, resulting in a stable agreement of the optical axis $L_2$ and the luminal central axis $L_0$.

Similarly, as shown in FIG. 31, a plurality of fins 212 along the longitudinal axis direction of the capsule endoscope 1 may be provided on an outer surface of the capsule housing 3 to stabilize the posture of the capsule endoscope 1a through straightening of the fluid 7.

Also, as shown in FIG. 32, a plurality of grooves 213 along the longitudinal axis direction of the capsule endoscope 1a may be provided for straightening.

Straighteners such as the fins 212 for straightening the flow of the fluid 7 are provided in the capsule endoscopes shown in FIG. 30 to FIG. 31, but the posture of the capsule endoscope 1a may also be stabilized by rotating the capsule endoscope about the longitudinal axis.

In a capsule endoscope shown in FIG. 33, a plurality of through holes 214 are provided near the surface of the capsule housing and the through holes 214 are formed to be spiral about the longitudinal axis. The capsule endoscope receives thereby fluid resistance by the fluid 7 passing through the through holes 214 so that the capsule endoscope rotates about the longitudinal axis. The capsule endoscope can attain through the rotational movement a stable physical relationship in which the longitudinal axis runs along the fluid.

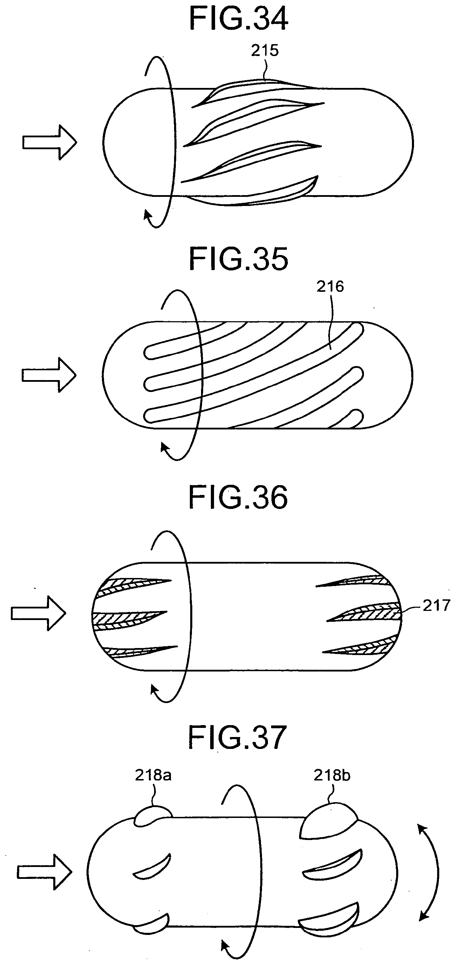

Instead of the through holes 214 shown in FIG. 33, fins 215, grooves 216, or finned cutouts 217 may be provided. Each of the fins 215, grooves 216, or finned cutouts 217 can stabilize the posture of the capsule endoscope by rotating the capsule endoscope about the longitudinal axis (see FIGS. 34 to 36).

Straighteners are provided or the capsule endoscope is rotated in the embodiment described above to stabilize the posture of the capsule endoscope, but the present invention is not limited to such modifications of the present embodiment and the longitudinal axis direction of the capsule endoscope may be forced to change bit by bit while roughly stabilizing the posture of the capsule endoscope.

For example, as shown in FIG. 37, a plurality of fins 218a and a plurality of fins 218b for receiving fluid resistance of the fluid 7 may be provided at different end edges in such a way that the fluid resistance received by the fins 218a is different from that received by the fins 218b to produce different turning efforts at both end edges, causing eccentric movement about the longitudinal axis center line $L_1$. By making this eccentric movement, images can be captured inside the lumen at substantially still wider angles.

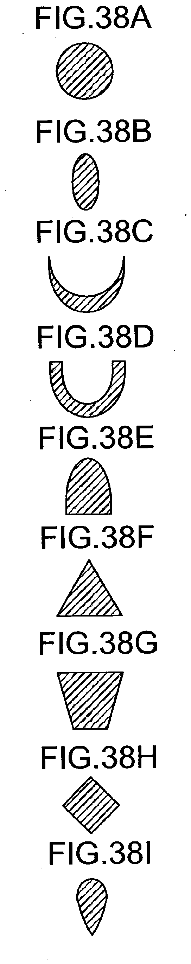

FIG. 38A to FIG. 38I show modifications of the sectional shape of the through holes, fins, grooves, and cutouts shown in FIG. 30 to FIG. 37. Each sectional shape can take any shape shown here or a combination of these shapes.

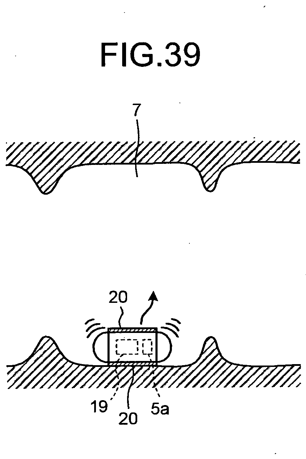

FIG. 39 shows a modification obtained by providing a vibrator 19 realized by an eccentric motor or the like, a control unit 5a for performing vibration control of the vibrator 19, and a contact recognizer 20 for sensing a contact state in which the capsule endoscope is in contact with a body cavity tissue in the above capsule endoscope. The whole capsule endoscope 1a is vibrated when no image is captured to make it easier to be separated from the wall surface of the lumen. Instead of the vibrator 19, a magnet may be provided. In this case, the control unit 5a is removed and the magnet may be caused to vibrate by applying an external oscillating magnetic field, thereby causing the capsule endoscope itself to vibrate. However, it is also preferable to perform a control operation such that the magnet is vibrated only when no image is captured. For example, the vibrator 19 is caused to vibrate when the contact recognizer 20 detects that the capsule endoscope is in contact with a body cavity wall. When the capsule endoscope separates from the body cavity wall due to the vibration and the contact recognizer 20 detects that the capsule endoscope is not in contact with a body cavity wall, the vibration of the vibrator 19 is stopped so that images can be captured. In this way, more stable images can be captured.

**Claims**

1. A capsule medical apparatus (1; 1a; 1b), comprising a capsule housing (3) having a protruded portion (lp) on a longitudinal axis at one end (3b) of the capsule housing (3), wherein
the protruded portion (lp) is formed so that an outer surface of the protruded portion (lp) intersects perpendicularly with a first straight line,
the first straight line substantially intersects with a second straight line (lvm) connecting a center of buoyancy (Pv) and a center of gravity (Pm) when the capsule housing (3) is in liquid (7) in a body cavity, and
the first straight line and the second straight line (lvm) substantially intersect at a point (Pf) where buoyant moment produced by buoyancy acting on the center of buoyancy and gravitational moment produced by gravity acting on the center of gravity are substantially balanced,
wherein the capsule medical apparatus (1; 1a; 1b) is in point contact (Pp) with a body cavity inner wall surface (2a) at an intersection of the first straight line that intersects perpendicularly with the outer surface of the protruded portion (lp) and the protruded portion (lp),
wherein either the point (Pf) at which the moments are balanced is positioned on a line between the point contact (Pp) and a center of curvature (Pc) of the protruded portion (lp) at the point contact (Pp) in a plane including the point (Pf) at which the moments are balanced, the point contract (Pp), and the center of buoyancy (Pv), or the point contract (Pp) is positioned on a line between the point (Pf) at which the moments are balanced and the center of curvature (Pc).

2. The capsule medical apparatus (1; 1a; 1b) according to claim 1, wherein specific gravity of the capsule medical apparatus (1; 1a; 1b) is a proximity value of specific gravity of the liquid (7).

3. The capsule medical apparatus (1; 1a; 1b) according to claim 1, wherein when the gravity is greater than the buoyancy, a distance (PfPv) between the point (Pf) at which the moments are balanced and the center of buoyancy (Pv) is larger than a distance (PfPm) between the point (Pf) at which the moments are balanced and the center of gravity (Pm), and the protruded portion (lp) is brought into contact with an inner wall (2a) of the body cavity downward in a vertical direction of the point (Pf) at which the moments are balanced.

4. The capsule medical apparatus (1; 1a; 1b) according to claim 1, wherein when the gravity is smaller as compared to the buoyancy, a distance (PfPv) between the point (Pf) at which the moments are balanced and the center of buoyancy (Pv) is smaller than a distance (PfPm) between the point (Pf) at which the moments and the center of gravity (Pm), and the protruded portion (lp) is brought into contact with an inner wall (2a) of the body cavity upward in a vertical direction of the point (Pf) at which the moments are balanced.

5. The capsule medical apparatus (1; 1a; 1b) according to claim 1, wherein an acute angle formed by the first straight line that intersects perpendicularly with the outer surface of the protruded portion (lp) and the longitudinal axis is equal to or smaller than 80 degrees.

6. The capsule medical apparatus (1; 1a; 1b) according to claim 1, wherein the capsule housing (3) includes an imaging system (4; 4a, 4b) on at the other end (3a) of the capsule housing (3), the imaging system (4; 4a, 4b) imaging an image in the body cavity.

7. The capsule medical apparatus (1; 1a; 1b) according to claim 6, wherein
the center of gravity (Pm) is positioned at an eccentric position from the longitudinal axis of the capsule housing (3), and
an imaging axis of the imaging system (4; 4a, 4b) is provided at an angle with respect to the longitudinal axis centering around a straight line perpendicular to a plane including the longitudinal axis and the center of gravity (Pm).

8. The capsule medical apparatus (1; 1a; 1b) according to claim 7, further comprising a magnetic body (72), within the capsule housing (3), having magnetism that is substantially perpendicular to the longitudinal axis, wherein
the capsule housing (3) turns about the longitudinal axis by rotating magnetic field applied from outside.

9. The capsule medical apparatus (1; 1a; 1b) according to claim 1, wherein the protruded portion (lp) includes a flat portion, an intersection of the first straight line that intersects perpendicularly with the outer surface of the protruded portion (lp) and the protruded portion (lp) is at the flat portion.

10. The capsule medical apparatus (1; 1a; 1b) according to claim 1, wherein the protruded portion (lp) includes a circular truncated cone plane portion, an intersection of the first straight line that intersects perpendicularly with the outer surface of the protruded portion (lp) and the protruded portion (lp) is at the circular truncated cone plane portion.

11. The capsule medical apparatus (1; 1a; 1b) according to claim 1, wherein the capsule housing (3) includes a body portion that is in a substantial cylindrical shape and whose center axis is parallel to the longitudinal axis, and
a radius of curvature of the protruded portion (lp) at an intersection of the first straight line that intersects perpendicularly with the outer surface of the protruded portion (lp) and the protruded portion (lp) is greater than a diameter of the substantial cylindrical shape.

**Patentansprüche**

1. Kapselförmige medizinische Vorrichtung (1; 1a; 1b) aufweisend ein Kapselgehäuse (3) mit einem in Richtung der Längsachse an einem Ende (3b) des Kapselgehäuses (3) vorgesehenen vorstehenden Abschnitt (lp) wobei
der vorstehende Abschnitt (lp) derart ausgebildet ist, dass eine Außenfläche des vorstehenden Abschnitts (lp) eine erste Gerade senkrecht schneidet,
die erste Gerade im Wesentlichen eine zweite Gerade (lvm) schneidet, die einen Auftriebsmittelpunkt (Pv) und einen Körperschwerpunkt (Pm) schneidet, wenn sich das Kapselgehäuse (3) in einer Flüssigkeit (7) in einer Körperhöhle befindet, und
die erste Gerade und die zweite Gerade (lvm) sich im Wesentlichen in einem Punkt (Pf) schneiden, an dem das Auftriebsmoment, das von dem an dem Auftriebsmittelpunkt wirkenden Auftrieb erzeugt wird, und das Gravitationsmoment, das von der an dem Körperschwerpunkt wirkenden Gravitation erzeugt wird, im Wesentlichen ausgeglichen sind,
wobei die kapselförmige medizinische Vorrichtung (1; 1a; 1b) in einem Punktkontakt (Pp) mit einer Innenwandfläche (2a) einer Körperhöhle an dem Schnittpunkt der ersten Gerade, die senkrecht die Außenfläche des vorstehenden Abschnitts (lp) schneidet, und dem vorstehenden Abschnitt (lp) steht,
wobei entweder der Punkt (Pf), an dem die Momente ausgeglichen sind, auf einer Linie zwischen dem Punktkontakt (Pp) und einem Krümmungsmittelpunkt (Pc) des vorstehenden Abschnitts (lp) an dem Punktkontakt (Pp) in einer Ebene positioniert ist, die den Punkt (Pf), an dem die Momente ausgeglichen sind, der Punktkontakt (Pp) und den Auftriebsmittelpunkt (Pv) enthält, oder der Punktkontakt (Pp) auf einer Linie zwischen dem Punkt (Pf), an dem die Momente ausgeglichen sind, und dem Krümmungsmittelpunkt (Pc) positioniert ist.

2. Kapselförmige medizinische Vorrichtung (1; 1a; 1b) nach Anspruch 1, wobei die spezifische Dichte der kapselförmige medizinischen Vorrichtung (1; 1a; 1b) ein Näherungswert der spezifischen Dichte der Flüssigkeit (7) ist.

3. Kapselförmige medizinische Vorrichtung (1; 1a; 1b) nach Anspruch 1, wobei dann, wenn die Gravitationskraft größer als der Auftrieb ist, ein Abstand (PfPv) zwischen dem Punkt (Pf), an dem die Momente ausgeglichen sind, und dem Auftriebsmittelpunkt (Pv) größer als ein Abstand (PfPm) zwischen dem Punkt (Pf), an dem die Momente ausgeglichen sind, und dem Körperschwerpunkt (Pm) ist, und der vorstehende Abschnitt in einer vertikalen Richtung unterhalb des Punkts (Pf), an dem die Momente ausgeglichen sind, in Kontakt mit einer Innenwand (2a) der Körperhöhle bringbar ist.

**4.** Kapselförmige medizinische Vorrichtung (1; 1a; 1b) nach Anspruch 1, wobei, dann, wenn die Gravitationskraft kleiner als der Auftrieb ist, ein Abstand (PfPv) zwischen dem Punkt (Pf), an dem die Momente ausgeglichen sind, und dem Auftriebsmittelpunkt (Pv) kleiner als ein Abstand (PfPm) zwischen dem Punkt (Pf), an dem die Momente ausgeglichen sind, und dem Körperschwerpunkt (Pm) ist, und der vorstehende Abschnitt (lp) in vertikaler Richtung oberhalb des Punkts (Pf) an dem die Momente ausgeglichen sind, mit der Innenwand (2a) der Körperhöhle in Kontakt bringbar ist.

**5.** Kapselförmige medizinische Vorrichtung (1; 1a; 1b) nach Anspruch 1, wobei ein von der ersten Gerade, die die Außenfläche des vorstehenden Abschnitts (lp) senkrecht schneidet, und der Längsachse gebildeter spitzer Winkel gleich oder kleiner 80 Grad ist.

**6.** Kapselförmige medizinische Vorrichtung (1; 1a; 1b) nach Anspruch 1, wobei das Kapselgehäuse (3) ein Bildsystem (4; 4a; 4b) an dem anderen Ende (3a) des Kapselgehäuses (3) umfasst, wobei das Bildsystem (4; 4a; 4b) ein Bild in der Körperhöhle aufnimmt.

**7.** Kapselförmige medizinische Vorrichtung (1; 1a; 1b) nach Anspruch 6, wobei
der Körperschwerpunkt (Pm) an einer exzentrischen Position von der Längsachse des Kapselgehäuses (3) positioniert ist, und
eine Bildachse des Bildsystems (4; 4a, 4b) an einem Winkel bezüglich der Längsachse vorgesehen ist, der sich um eine gerade Linie erstreckt, die senkrecht zu einer die Längsachse und den Körperschwerpunkt (Pm) umfassenden Ebene erstreckt.

**8.** Kapselförmige medizinische Vorrichtung (1; 1a; 1b) nach Anspruch 7, ferner aufweisend einen magnetischen Körper (72) innerhalb des Kapselgehäuses (3) mit einem Magnetismus, der sich im Wesentlichen senkrecht zur Längsachse erstreckt, wobei
das Kapselgehäuse (3) sich aufgrund eines von außen ausgeübten, sich drehenden drehenden Magnetfelds um die Längsachse dreht.

**9.** Kapselförmige medizinische Vorrichtung (1; 1a; 1b) nach Anspruch 1, wobei der vorstehende Abschnitt (lp) einen flachen Abschnitt umfasst, wobei ein Schnittpunkt der ersten Gerade, die die Außenfläche des vorstehenden Abschnitts (lp) senkrecht schneidet, und dem vorstehenden Abschnitt (lp) sich an dem flachen Abschnitt befindet.

**10.** Kapselförmige medizinische Vorrichtung (1; 1a; 1b) nach Anspruch 1, wobei der vorstehende Abschnitt (lp) einen kreisförmigen, kegelstumpfartigen, ebenen Abschnitt umfasst, wobei ein Schnittpunkt der ersten Gerade, die die Außenfläche des vorstehenden Abschnitts (lp) senkrecht schneidet, und dem vorstehenden Abschnitt (lp) sich an dem kreisförmigen, kegelstumpfartigen, ebenen Abschnitt befindet.

**11.** Kapselförmige medizinische Vorrichtung (1; 1a; 1b) nach Anspruch 1, wobei das Kapselgehäuse (3) einen Körperabschnitt umfasst, der im Wesentlichen eine zylindrische Form aufweist und dessen Mittelachse sich parallel zur Längsachse erstreckt, und
ein Krümmungsradius des vorstehenden Abschnitts (lp) an einem Schnittpunkt der ersten Gerade, die die Außenfläche des vorstehenden Abschnitts (lp) senkrecht schneidet, und dem vorstehenden Abschnitt (lp) größer als ein Durchmesser der im Wesentlichen zylindrischen Form ist.

**Revendications**

**1.** Appareil médical de type capsule (1 ; 1a ; 1b), comprenant un logement (3) de capsule ayant une partie saillante (lp) sur un axe longitudinal à une extrémité (3b) du logement (3) de capsule, dans lequel
la partie saillante (lp) est formée de telle manière qu'une surface extérieure de la partie saillante (lp) se coupe perpendiculairement avec une première ligne droite,
la première ligne droite se coupe sensiblement avec une deuxième ligne droite (lvm) connectant un centre de flottabilité (Pv) et un centre de gravité (Pm) lorsque le logement (3) de capsule est dans un liquide (7) dans une cavité corporelle, et
la première ligne droite et la deuxième ligne droite (lvm) se coupent sensiblement en un point (Pf) où un moment de flottabilité produit par une flottabilité agissant sur le centre de flottabilité et un moment gravitationnel produit par la gravité agissant sur le centre de gravité sont sensiblement équilibrés,
dans lequel l'appareil médical de type capsule (1 ; la ; 1b) est en contact par point (Pp) avec une surface (2a) de paroi intérieure de cavité corporelle à une intersection de la première ligne droite qui se coupe perpendiculairement avec la surface extérieure de la partie saillante (lp) et de la partie saillante (lp),

dans lequel soit le point (Pf) auquel les moments sont équilibrés est positionné sur une ligne entre le contact par point (Pp) et un centre de courbure (Pc) de la partie saillante (lp) au contact par point (Pp) dans un plan incluant le point (Pf) auquel les moments sont équilibrés, le contact par point (Pp) et le centre de flottabilité (Pv), soit le contact par point (Pp) est positionné sur une ligne entre le point (Pf) auquel les moments sont équilibrés et le centre de courbure (Pc).

2. Appareil médical de type capsule (1 ; 1a ; 1b) selon la revendication 1, dans lequel le poids spécifique de l'appareil médical de type capsule (1 ; 1a ; 1b) est une valeur à proximité du poids spécifique du liquide (7).

3. Appareil médical de type capsule (1 ; 1a ; 1b) selon la revendication 1, dans lequel, lorsque la gravité est supérieure à la flottabilité, une distance (PfPv) entre le point (Pf) auquel les moments sont équilibrés et le centre de flottabilité (Pv) est plus grande qu'une distance (PfPm) entre le point (Pf) auquel les moments sont équilibrés et le centre de gravité (Pm), et la partie saillante (lp) est amenée en contact avec une paroi intérieure (2a) de la cavité corporelle vers le bas dans un sens vertical du point (Pf) auquel les moments sont équilibrés.

4. Appareil médical de type capsule (1 ; 1a ; 1b) selon la revendication 1, dans lequel, lorsque la gravité est plus petite par rapport à la flottabilité, une distance (PfPv) entre le point (Pf) auquel les moments sont équilibrés et le centre de flottabilité (Pv) est plus petite qu'une distance (PfPm) entre le point (Pf) auquel les moments sont équilibrés et le centre de gravité (Pm), et la partie saillante (lp) est amenée en contact avec une paroi intérieure (2a) de la cavité corporelle vers le haut dans un sens vertical du point (Pf) auquel les moments sont équilibrés.

5. Appareil médical de type capsule (1 ; 1a ; 1b) selon la revendication 1, dans lequel un angle aigu formé par la première ligne droite qui se coupe perpendiculairement avec la surface extérieure de la partie saillante (lp) et l'axe longitudinal est égal ou inférieur à 80 degrés.

6. Appareil médical de type capsule (1 ; 1a ; 1b) selon la revendication 1, dans lequel le logement (3) de capsule inclut un système d'imagerie (4 ; 4a, 4b) à l'autre extrémité (3a) du logement (3) de capsule, le système d'imagerie (4 ; 4a, 4b) imageant une image dans la cavité corporelle.

7. Appareil médical de type capsule (1 ; 1a ; 1b) selon la revendication 6, dans lequel
le centre de gravité (Pm) est positionné en une position excentrée par rapport à l'axe longitudinal du logement (3) de capsule, et
un axe d'imagerie du système d'imagerie (4 ; 4a, 4b) est prévu à un angle par rapport à l'axe longitudinal se centrant autour d'une ligne droite perpendiculaire à un plan incluant l'axe longitudinal et le centre de gravité (Pm).

8. Appareil médical de type capsule (1 ; 1a ; 1b) selon la revendication 7, comprenant en outre un corps magnétique (72), à l'intérieur du logement (3) de capsule, ayant un magnétisme qui est sensiblement perpendiculaire à l'axe longitudinal, dans lequel
le logement (3) de capsule tourne autour de l'axe longitudinal par rotation d'un champ magnétique appliqué depuis l'extérieur.

9. Appareil médical de type capsule (1 ; 1a ; 1b) selon la revendication 1, dans lequel la partie saillante (lp) inclut une partie plate, une intersection de la première ligne droite qui se coupe perpendiculairement avec la surface extérieure de la partie saillante (lp) et de la partie saillante (lp) est au niveau de la partie plate.

10. Appareil médical de type capsule (1 ; 1a ; 1b) selon la revendication 1, dans lequel la partie saillante (lp) inclut une partie plane en cône tronqué circulaire, une intersection de la première ligne droite qui se coupe perpendiculairement avec la surface extérieure de la partie saillante (lp) et de la partie saillante (lp) est au niveau de la partie plane en cône tronqué circulaire.

11. Appareil médical de type capsule (1 ; 1a ; 1b) selon la revendication 1, dans lequel le logement (3) de capsule inclut une partie de corps qui est à une forme sensiblement cylindrique et dont l'axe central est parallèle à l'axe longitudinal, et
un rayon de courbure de la partie saillante (lp) à une intersection de la première ligne droite qui se coupe perpendiculairement avec la surface extérieure de la partie saillante (lp) et de la partie saillante (lp) est supérieur à un diamètre de la forme sensiblement cylindrique.

# FIG.1

# FIG.2

# FIG.3

# FIG.4

# FIG.5

# FIG.6

# FIG.7

# FIG.8

3b

3a

E1

# FIG.9

E2 E2

# FIG.10

E3 E3

# FIG.11

3b

3a

E4

# FIG.12

11

# FIG.13

12

# FIG.14

13

# FIG.15

14

# FIG.16

# FIG.17

# FIG.18

# FIG19

EP 2 101 630 B1

# FIG.20

# FIG.21

# FIG.22

# FIG.23

## FIG.24

## FIG.25

# FIG.26

## FIG.27A

## FIG.27B

# FIG.28

# FIG.29

# FIG.30

211

# FIG.31

212

# FIG.32

213

# FIG.33

214

# FIG.34

215

# FIG.35

216

# FIG.36

217

# FIG.37

218a      218b

FIG.38A

FIG.38B

FIG.38C

FIG.38D

FIG.38E

FIG.38F

FIG.38G

FIG.38H

FIG.38I

# FIG.39

**EP 2 101 630 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0295351 A

- WO 2005060348 A